(19) Europäisches Patentamt
European Patent Office
Office européen des brevets

(11) EP 1 454 980 A2

(12) EUROPEAN PATENT APPLICATION

(43) Date of publication:
08.09.2004 Bulletin 2004/37

(51) Int Cl.[7]: **C12N 1/20**, C02F 3/34

(21) Application number: 04004958.7

(22) Date of filing: 03.03.2004

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **03.03.2003 JP 2003056074**

(71) Applicants:
- **EBARA CORPORATION**
  **Ohta-ku, Tokyo (JP)**
- **JAPAN SEWAGE WORKS AGENCY**
  **Tokyo (JP)**

(72) Inventors:
- **Morita, Tomoyuki**
  **Kawasaki-shi Kanagawa-ken (JP)**
- **Onda, Kensuke**
  **Fujisawa-shi Kanagawa-ken (JP)**
- **Miya, Akiko**
  **Kamakura-shi Kanagawa-ken (JP)**
- **Tada, Keitaro**
  **Zushi-shi Kanagawa-ken (JP)**
- **Hashimoto, Toshikazu**
  **Toda-shi Saitama-ken (JP)**
- **Mishina, Fumio**
  **Saitama-shi Saitama-ken (JP)**

(74) Representative: **HOFFMANN - EITLE**
**Patent- und Rechtsanwälte**
**Arabellastrasse 4**
**81925 München (DE)**

# (54) Female sex hormone degrading bacteria and use thereof

(57) The present invention provides female sex hormone degrading bacteria belonging to the genus Novosphingobium, Sphingomonas or Ochrobactrum and having at least one of the property (a) the ability to degrade at least ethynylestradiol; the property (b) the ability to degrade at least 17β-estradiol and/or estrone, and having a degradation rate constant $k_1$ for 17β-estradiol and/or estrone of 0.05 or more; and the property (c) the ability to degrade at least 17β-estradiol and/or estrone, and having the ability to decrease the concentration of 17β-estradiol and/or estrone, contained in wastewater, to less than 10 ng/L. These bacteria can efficiently degrade various female sex hormones, including recalcitrant synthetic female sex hormones, to such very low concentrations as not to affect the environment, and can easily be cultured and proliferated.

EP 1 454 980 A2

SEQUENCE LISTING

<110> Ebara Corporation / Japan Sewage Works Agency

<120> Female Hormone Degrading Bacteria and the use thereof

<130> EP/E-6-842

<160> 5

<210> 1
<211> 1486
<212> DNA
<213> Novosphingobium sp. JEM-1

<400> 1
agagtttgat cctgggctca gaacgaacgc tgggcggcat gcctaacaca tgcaagtcga 60
acgaagcctt cgggcttagt ggcgcacggg tgcgtaacgc gtgggaacct gcctttaggt 120
tcggaataac acagagaaat ttgtgctaat accggatgat gtcttcggac caaagattta 180
tcgcctttag atgggcccgc gttggattag ctagttggta gggtaaaggc ctaccaagvc 240
gacgatccat agctggtctg agaggatgat cagccacact gggactgaga cacggcccag 300
actcctacgg gaggcagcag tggggaatat tggacaatgg gcgaaagcct gatccagcaa 360
tgccgcgtga gtgatgaagg ccttagggtt gtaaagctct ttaccaggg atgataatga 420
cagtacctgg agaataagct ccggctaact ccgtgccagc agccgcggta atacggaggg 480
agctagcgtt gttcggaatt actgggcgta aagcgcgcgt aggcggctat tcaagtcagg 540
ggtgaaatcc cggggctcaa ccccggaact gcctttgaaa ctgggtagct agaatcctgg 600
agaggcgagt ggaattccga gtgtagaggt gaaattcgta gatattcgga agaacaccag 660
tggcgaaggc gactcgctgg acaggtattg acgctgaggt gcgaaagcgt ggggagcaaa 720
caggattaga taccctggta gtccacgccg taaacgatga taactagctg tccgggttca 780
tggaacttgg gtggcgcagc taacgcatta agttatccgc ctggggagta cggtcgcaag 840
attaaaactc aaaggaattg acgggggcct gcacaagcgg tggagcatgt ggtttaattc 900
gaagcaacgc gcagaacctt accagcgttt gacatcctga tcgcggatta gagagatctt 960
ttccttcagt tcggctggat cagtgacagg tgctgcatgg ctgtcgtcag ctcgtgtcgt 1020
gagatgttgg gttaagtccc gcaacgagcg caaccctcgt ccttagttgc catcattcag 1080
ttgggcactc taaggaaacc gccggtgata agccggagga aggtggggat gacgtcaagt 1140
cctcatggcc cttacacgct gggctacaca cgtgctacaa tggcggtgac agtgggcagc 1200

aacctcgcga ggggtagcta atctccaaaa accgtctcag ttcggattgt tctctgcaac 1260
tcgagagcat gaaggcggaa tcgctagtaa tcgcggatca gcatgccgcg gtgaatacgt 1320
tcccaggcct tgtacacacc gcccgtcaca ccatgggagt tgggttcacc cgaaggcgtc 1380
gcgctaaccc gcaagggagg caggcgacca cggtgggctt agcgactggg gtgaagtcgt 1440
aacaaggtag ccgtagggga acctgcggct ggatcacctc ctttct 1486

<210> 2
<211> 1427
<212> DNA
<213> Sphingomonas sp. JEM-2

<400> 2
cgtgcctcag aacgaacgct gccggcatgc ctaacacatg caagtcgaac gaaggcttcg 60
gccttagtgg cgcacgggtg cgtaacgcgt gggaatctgc ccttgggtac ggaataacag 120
tgagaaatta ctgctaatac cgtatgatga cgtaagtcca aagatttatc gcccaaggat 180
gagcccgcgt aggattagct agttggtgag gtaaaggctc accaaggcga cgatccttag 240
ctggtctgag aggatgatca gccacactgg gactgagaca cggcccagac tcctacggga 300
ggcagcagtg gggaatattg gacaatgggc gaaagcctga tccagcaatg ccgcgtgagt 360
gatgaaggcc ctagggttgt aaagctcttt tacccgggaa gataatgact gtaccgggag 420
aataagcccc ggctaactcc gtgccagcag ccgcggtaat acggaggggg ctagcgttgt 480
tcggaattac tgggcgtaaa gcgtacgtag gcggctttgt aagttagagg tgaaagcccg 540
gggctcaacc ccggaattgc ctttaagact gcatcgcttg aacgtcggag aggtgagtgg 600
aattccgagt gtagaggtga aattcctaga tattcggaag aacaccagtg gcgaacggg 660
gctcactgga cgactgttga cgctgaggta cgaaagcgtg gggagcaaac aggattagat 720
accctggtag tccacgccgt aaacgatgat aactagctgt ccgggcactt ggtgcttggg 780
tggcgcagct aacgcattaa gttatccgcc tggggagtac ggccgcaagg ttaaaactca 840
aagaaattga cgggggcctg cacaagcggt ggagcatgtg gtttaattcg aagcaacgcg 900
cagaacctta ccaacgtttg acatccctat cgcggttacc agagatggtt tccttcagtt 960
cggctggata ggtgacaggt gctgcatggc tgtcgtcagc tcgtgtcgtg agatgttggg 1020
ttaagtcccg caacgagcgc aaccctcgcc tttagttgcc atcatttagt tgggtactct 1080
aaaggaaccg ccggtgataa gccggaggaa ggtggggatg acgtcaagtc ctcatggccc 1140
ttacgcgttg ggctacacac gtgctacaat ggcaactaca gtgggcagca aactcgcgag 1200
ggtgagctaa tctccaaaag ttgtctcagt tcggattgtt ctctgcaact cgagagcatg 1260
aaggcggaat cgctagtaat cgcggatcag catgccgcgg tgaatacgtt cccaggcctt 1320
gtacacaccg cccgtcacac catgggagtt ggattcaccc gaaggcgctg cgctcaccgc 1380
aagggggcag gcgaccacgg tgggtttagc gactggggtg aaatcgt 1427

<210> 3
<211> 1416
<212> DNA
<213> Sphingomonas sp. JEM-3

<400> 3
cctgtctcag aacgaacgct ggcggcatgc ctaacacatg caagtcgaac gaaggcttcg 60
gccttagtgg cgcacgggtg cgtaacgcgt gggaatctgc ccttaggtac ggaataacag 120
tgagaaatta ctgctaatac cgtatgatgt cgcaagacca aagatttatc gcctaaggat 180
gagcccgcgt aggattagct agttggtgag gtaaaagctc accaaggcga cgatccttag 240
ctggtctgag aggatgatca gccacactgg gactgagaca cggcccagac tcctacggga 300
ggcagcagtg gggaatattg gacaatgggc gaaagcctga tccagcaatg ccgcgtgagt 360
gatgaaggcc ttagggttgt aaagctcttt tacccgggaa gataatgact gtaccgggag 420
aataagcccc ggctaactcc gtgccagcag ccgcggtaat acggaggggg ctagcgttgt 480
tcggaattac tgggcgtaaa gcgtacgtag gcggctttgt aagttagagg tgaaagcccg 540
gggctcaacc ccggaattgc ctttaagact gcatcgcttg aacgtcggag aggtgagtgg 600
aattccgagt gtagaggtga aattcgtaga tattcggaag aacaccagtg gcgaaggcgg 660
ctcactggac gactgttgac gctgaggtac gaaagcgtgg ggagcaaaca ggattagata 720
ccctggtagt ccacgccgta aacgatgata actagctgtc cgggtacttg gtacttgggt 780
ggcgcagcta acgcattaag ttatccgcct ggggagtacg gccgcaaggt taaaactcaa 840
agaaattgac gggggcctgc acaagcggtg gagcatgtgg tttaattcga agcaacgcgc 900
agaaccttac caacgtttga catccctatc gcggttacca gagatggttt ccttcagttc 960
ggctggatag gtgacaggtg ctgcatggct gtcgtcagct cgtgtcgtga gatgttgggt 1020
taagtcccgc aacgagcgca accctcgcct ttagttgcca tcatttagtt gggcactcta 1080
aaggaaccgc cggtgataag ccggaggaag gtggggatga cgtcaagtcc tcatggccct 1140
tacgcgttgg gctacacacg tgctacaatg gcaactacag tgggcagcaa gccggcgacg 1200
gtgagctaat ctccaaaagt tgtctcagtt cggattgttc tctgcaactc gagagcatga 1260
aggcggaatc gctagtaatc gcggatcagc atgccgcggt gaatacgttc ccaggccttg 1320
tacacaccgc ccgtcacacc atgggagttg gattcacccg aaggccctgc gctaacccgc 1380
aagggaggca ggcgaccacc gtgggtttag cgaatg 1416

<210> 4
<211> 1462
<212> DNA
<213> Sphingomonas sp. JEM-14

<400> 4

cctggctcag aacgaacgct ggcggcatgc ctaacacatg caagtcgaac gagatcttcg 60
gatctagtgg cgcacgggtg cgtaacgcgt gggaatctac ccttgggttc ggaataacag 120
ttagaaatga ctgctaatac cggatgatga cgtaagtcca aagatttatc gcccaaggat 180
gagcccgcgt aggattaggt agttggtgag gtaaaggctc accaagccga cgatccttag 240
ctggtctgag aggatgatca gccacactgg gactgagaca cggcccagac tcctacggga 300
ggcagcagtg gggaatattg gacaatgggc gaaagcctga tccagcaatg ccgcgtgagt 360
gatgaaggcc ttagggttgt aaagctcttt tacccgtgat gataatgaca gtagcgggag 420
aataagctcc ggctaactcc gtgccagcag ccgcggtaat acggagggag ctagcgttgt 480
tcggaattac tgggcgtaaa gcgcacgtag gcggctttgt aagttagagg tgaaagcctg 540
gagctcaact ccagaactgc ctttaagact gcatcgcttg aatccaggag aggtgagtgg 600
aattccgagt gtagaggtga aattcgtaga tattcggaag aacaccagtg gcgaaggcgg 660
ctcactggac tggtattgac gctgaggtgc gaaagcgtgg ggagcaaaca ggattagata 720
ccctggtagt ccacgccgta aacgatgata actagctgtc tgggtgcatg gcacttaggt 780
ggcgcagcta acgcattaag ttatccgcct ggggagtacg gtcgcaagat taaaactcaa 840
aggaattgac gggggcctgc acaagcgggt ggagcatgtg gtttaattcg aagcaacgcg 900
cagaacctta ccagcgtttg acatgccggt cgcggatcgt ggagacactt tccttcagtt 960
cggctggacc gtgcacaggt gctgcatggc tgtcgtcagc tcgtgtcgtg agatgttggg 1020
ttaagtcccg caacgagcgc aaccctcgtc cttagttgcc atcattcagt tgggcactct 1080
aaggaaaccg ccggtgataa gccggaggaa ggtggggatg acgtcaagtc ctcatggccc 1140
ttacgcgctg ggctacacac gtgctacaat ggcaactaca gtgggcagcg aactcgcgag 1200
ggtaagctaa tctccaaaag ttgtctcagt tcggattgtt ctctgcaact cgagagcatg 1260
aaggcggaat cgctagtaat cgcggatcag catgccgcgg tgaatacgtt cccaggcctt 1320
gtacacaccg cccgtcacac catgggagtt ggattcactc gaaggcgttg agctaacccg 1380
caagggaggc aggcgaccac agtgggttta gcgactgggg tgaagtcgta acaaggtagc 1440
cgtaggggaa cctgcggatg ga 1462

<210> 5
<211> 1440
<212> DNA
<213> Ochrobactrum sp. JEM-23

<400> 5

aatttttttt tttccttggc tcagaacgaa cgctggcggc aggcttaaca catgcaagtc 60
gagcgccccg caaggggagc ggcagacggg tgagtaacgc gtgggaatct accttttgct 120

```
acggaacaac agttggaaac gactgctaat accgtatgtg cccttcgggg gaaagattta 180
tcggcaaagg atgagcccgc gttggattag ctagttggtg aggtaaaggc tcaccaaggc 240
gacgatccat agctggtctg agaggatgat cagccacact gggactgaga cacggcccag 300
actcctacgg gaggcagcag tggggaatat tggacaatgg gcgcaagcct gatccagcca 360
tgccgcgtga gtgatgaagg ccctagggtt gtaaagctct ttcaccggtg aagataatga 420
cggtaaccgg agaagaagcc ccggctaact tcgtgccagc agccgcggta atacgaaggg 480
ggctagcgtt gttcggattt actgggcgta aagcgcacgt aggcggattt ttaagtcagg 540
ggtgaaatcc cggggctcaa ccccggaact gcctttgata ctggaagtct tgagtatggt 600
agaggtgagt ggaattccga gtgtagaggt gaaattcgta gatattcgga ggaacaccag 660
tggcgaaggc ggctcactgg accattactg acgctgaggt gcgaaagcgt ggggagcaaa 720
caggattaga taccctggta gtccacgccg taaacgatga atgttagccg tcggggagtt 780
tactcttcgg tggcgcagct aacgcattaa acattccgcc tggggagtac ggtcgcaaga 840
ttaaaactca aaggaattga cgggggcccg cacaagcggt ggagcatgtg gtttaattcg 900
aagcaacgcg cagaacctta ccagcccttg acataccggt cgcggacaca gagatgtgtc 960
tttcagttcg gctggaccgg atacaggtgc tgcatggctg tcgtcagctc gtgtcgtgag 1020
atgttgggtt aagtcccgca acgagcgcaa ccctcgcctt tagttgccat catttagttg 1080
ggcactctaa agggactgcc agtgataagc tggaggaagg tggggatgac gtcaagtcct 1140
catggccctt acgggctggg ctacacacgt gctacaatgg tggtgacagt gggcagcaag 1200
cacgcgagtg tgagctaatc tccaaaagcc atctcagttc ggattgcact ctgcaactcg 1260
agtgcatgaa gttggaatcg ctagtaatcg cggatcagca tgccgcggtg aatacgttcc 1320
cgggccttgt acacaccgcc cgtcacacca tgggagttgg ttctgcccga aggcactgtg 1380
ctaaccgtaa ggaggcaggt gaccacggta gggtcagcga ctggggtgaa gtcgtaacaa 1440
```

## Description

BACKGROUND OF THE INVENTION

This invention relates to novel microorganisms which degrade female sex hormones, and water and waste treatment methods utilizing them.

In recent years, adverse effects, which female sex hormones of the mammalian origin present in sewage and wastewater exert on the environment, have been pointed out. In areas around rivers where sewage and its treated water flow in, for example, signs of feminization of male fish, such as increased blood vitellogenin levels and ovarian follicle formation, have been confirmed. These phenomena are actually confirmed as signs of feminization in male fish bred in clear water incorporating 17β-estradiol or estrone at concentrations of as low as about 10 ng/L. In many rivers, 17β-estradiol and estrone have been detected at extremely low concentrations of about 1 ng/L or in higher concentration ranges than them. In sewage and wastewater from stock farms, 17β-estradiol is detected at concentrations of 100 ng/L or more. In treated sewage discharges as well, 17β-estradiol or estrone is detected at concentrations of 10 ng/L or above. Synthetic female sex hormones, such as ethynylestradiol which is the active ingredient of pills, are also detected in domestic wastewater or environmental water.

As noted above, concern is expressed over the adverse influences of natural or synthetic female sex hormones on ecosystems in aquatic environments such as rivers and lakes. Thus, techniques for removing female sex hormones, which exist in sewage and stockbreeding-associated wastewater, to very low concentrations of the order of ng/L or less have been needed.

It has already been confirmed that treatment techniques, such as ozonation, advanced oxidation, activated carbon adsorption, and reverse osmosis membrane separation, are effective as techniques for removing female sex hormones from those aquatic environments. However, such techniques are applied as advanced treatment methods intended for recycling of treated water at some sewage treatment plants. Treatment of all of a vast amount of secondary treated sewage, which can contain traces of female sex hormones, by such advanced treatment methods, would be too inefficient. These advanced methods also require huge energy and high costs, and thus are not realistic solutions.

Compared with the above-described techniques, biological treatments, such as the activated sludge process, are low in cost, and can cope with a vast amount to be treated. Hence, it has been earnestly desired to provide techniques capable of decreasing female sex hormones contained in aquatic environments, such as 17β-estradiol, estrone, estriol or ethynylestradiol, to such sufficiently low levels as not to affect ecosystems such as fish, by biological treatments utilizing microorganisms.

Studies of publicly known biological treatment such as the activated sludge process, however, have shown that adequate action has not been taken for various female sex hormones at issue.

For example, they show low removal rates for natural female sex hormones such as 17β-estradiol, and estrone, which is the oxidized form of 17β-estradiol, is apt to remain in biological treated water, as compared with 17β-estradiol. Estrone, in particular, has the second highest estrogenic activity after 17β-estradiol. Thus, action to deal with estrone as well as 17β-estradiol is required. Ethynylestradiol, a synthetic female sex hormone, has high estrogenic activity comparable to that of natural female sex hormones, and should also be coped with. Its degradability is lower than that of natural female sex hormones.

Female sex hormone degrading microorganisms, present particularly in activated sludge, etc., have a limitation in female sex hormone concentrations for which they can show their degrading activity. They are deemed not to be sufficiently effective for decreasing these concentrations to those exerting no influence on the environment. For example, the concentrations of female sex hormones unaffecting to fish are generally very low concentrations of the order of ng/L. However, the phenomenon that these degradations do not proceed in such an extremely low concentration range has been confirmed in activated sludge treatment experiments on 17β-estradiol. It was actually found that many degrading microorganisms, isolated by the inventors, became very slow in these degradation rates at low concentrations of about 10 ng/L and, thereafter, their degrading reaction scarcely proceeded.

For example, a study of information on the degrading ability of Novosphingobium ARI-1 strain, a female sex hormone degrading microorganism, which is described in a non-patent document 1 (Applied Environmental Microbiology Apr. 2002. p2057-2060), shows the following facts: The ARI-1 strain degrades and removes about 60% of 17β-estradiol at a considerably high concentration of 1 g/L over the course of 20 days, but it was unknown whether this strain could remove the hormone down to an extremely low concentration of 1 ng/L. The ARI-1 strain also involves the problems that its degradation rate for estrone, which often remains in biological treated water, is low compared with that for 17β-estradiol, and that the ARI-1 strain cannot degrade ethynylestradiol.

According to information on the female sex hormone degrading microorganism described in a non-patent document 2 (abstracts of oral presentations at the 5th Annual Meeting of the Japan Society of Endocrine Disrupters Research, 2002, page 432), this microorganism is reported to cause degradation merely at high concentrations of 100 mg/L (100 ppm), and requires at least 24 hours until complete degradation of 17β-estradiol.

Generally, many microorganisms, which degrade difficultly degradable or recalcitrant organic matter, are slow in growth rate. This point may be a factor which restricts the industrial applicability of these microorganisms. Thus, it is important for even the above-mentioned microorganisms for use in treatment to be able to secure a high growth rate in ordinary nutrient media.
With the above-described background, there is a demand for the discovery of microorganisms which can efficiently degrade various female sex hormones, including recalcitrant synthetic female sex hormones, to such very low concentrations as not to affect the environment, and which can easily be cultured and proliferated.

SUMMARY OF THE INVENTION

To solve the foregoing problems, we, the inventors, conducted in-depth studies in an attempt to discover microorganisms which have high substrate affinity for very low concentrations of female sex hormones and which show high degradation rates. As a result, we succeeded in isolating a plurality of bacteria, which degrade various female sex hormones in waste water more efficiently than publicly known microorganisms, from activated sludge of sewage treatment facilities. This success led us to accomplish the present invention.
In an aspect, the present invention provides female sex hormone degrading bacteria belonging to the genus Novosphingobium, the genus Sphingomonas or the genus Ochrobactrum and having at least one of the following properties (a) to (c):

(a) Having the ability to degrade at least ethynylestradiol among female sex hormones;
(b) Having the ability to degrade at least 17β-estradiol and/or estrone among female sex hormones, and having a degradation rate constant $k_1$ for 17β-estradiol and/or estrone of 0.05 or more; and
(c) Having the ability to degrade at least 17β-estradiol and/or estrone among female sex hormones, and having the ability to decrease the concentration of 17β-estradiol and/or estrone, contained in an object to be treated, such as wastewater, to less than 10 ng/L.

In connection with the property (a), the bacteria of the present invention include bacteria having the property (a) and having the ability to further degrade 17β-estradiol, estrone and estriol.
In connection with the property (b), the bacteria of the present invention include bacteria having the property (b) and having, preferably, a degradation rate constant $k_1$ for 17β-estradiol and/or estrone of 0.5 or more, and more preferably, a degradation rate constant $k_1$ for estrone of 1.5 or more.
In connection with the property (c), the bacteria of the present invention include bacteria having the property (c) and having the ability to decrease the concentration of 17β-estradiol and/or estrone from about 10 μg/L to less than about 10 ng/L, preferably, to about 1 ng/L. Moreover, the bacteria of the present invention include bacteria having the property (c) and having the ability to achieve the concentration decrease in 24 hours at most, preferably in 3 hours, at their proper cell concentration.
In addition, the bacteria of the present invention include bacteria having a maximum specific growth rate ($\mu$max) of at least 0.1 when cultured in a nutrient medium.
Preferred embodiments of the bacteria according to the present invention are Novosphingobium sp. JEM-1 strain (FERM P-19234;BP-08637), Sphingomonas sp. JEM-3 strain (FERM P-19235;BP-08638), Sphingomonas sp. JEM-14 strain (FERM P-19236;BP-08639), and Ochrobactrum sp. JEM-23 strain (FERM P-19237;BP-08640). These deposited bacteria have at least one of the above-mentioned properties or characteristics, or have them in various combinations. The present specification is intended to disclose the bacteriological properties or characteristics of these bacteria in every combination.
Based on the above discovery, we developed a technique for efficiently decreasing various female sex hormones to very low concentrations by using either the isolated bacteria alone or with the bacteria coexistent in activated sludge.
In another aspect, therefore, the present invention provides a method for degradation treatment of a female sex hormone, which comprises bringing the bacterial cells having any of the above-described characteristics, or a carrier having the bacteria immobilized thereon, into contact with the female sex hormone.
The method of degradation treatment according to the present invention includes a method for degradation treatment of a female sex hormone in wastewater, which comprises passing the wastewater through the reactor including a carrier having carried thereon the bacteria having any of the above characteristics.
In the treatment method according to the present invention, the female sex hormone to be removed is, preferably, ethynylestradiol, 17β-estradiol, estrone and/or estriol.
The degradation treatment method of the present invention is disclosed as being aimed for a treatment method which utilizes the bacteria having the aforementioned characteristics or every combination of such bacteria.
In still another aspect, the present invention provides a female sex hormone degrading agent containing the bacteria having any such characteristics, or cultures of the bacteria.

BRIEF DESCRIPTION OF THE DRAWINGS

The present invention will become more fully understood from the detailed description given hereinbelow and the accompanying drawings which are given by way of illustration only, and thus are not limitative of the present invention, and wherein:

FIG. 1 shows changes in the concentration of 17β-estradiol in batch treatment experiments using the bacteria of the Example;
FIG. 2 shows changes in the concentration of estrone in batch treatment experiments using the bacteria of the Example;
FIG. 3 shows a phylogenetic tree prepared by comparing DNA sequences of the 16S-rRNA gene of Novosphingobium sp. JEM-1 strain, Sphingomonas sp. JEM-3 strain, Sphingomonas sp. JEM-14 strain, and Ochrobactrum sp. JEM-23 strain of the Example, and publicly known related bacteria;
FIG. 4 shows the growth curves of the respective degrading bacteria in culture media using 17β-estradiol as a carbon source;
FIG. 5 shows the growth curves of the respective degrading bacteria in culture media using estrone as a carbon source;
FIG. 6 shows the growth curves of Novosphingobium sp. JEM-1 strain at respective temperatures;
FIG. 7 shows the growth curves of Sphingomonas sp. JEM-3 strain at respective temperatures;
FIG. 8 shows the growth curves of Ochrobactrum sp. JEM-23 strain at respective temperatures;
FIG. 9 shows the female sex hormone treating effect of Novosphingobium sp. JEM-1 strain added to activated sludge; and
FIG. 10 shows the female sex hormone treating effect of Sphingomonas sp. JEM-3 strain added to activated sludge.

DETAILED DESCRIPTION OF THE PREFERRED EMBODIMENTS

(1) Isolation method of female sex hormone degrading microorganisms

According to the present invention, there are provided bacteria which can decrease various female sex hormones to very low concentrations, thereby substantially eliminating them. Herein, the female sex hormones refer to 17β-estradiol, estrone, estriol, 17α-estradiol, ethynylestradiol, conjugates of them, or female sex hormone-like substances similar to them. In the present invention, the female sex hormone means at least one substance selected from these substances, or any combination of them.

The isolation method of the female sex hormone degrading microorganisms is as follows: A source for isolation, such as activated sludge or soil, is cultured in a culture medium containing 17β-estradiol or estrone as a main carbon source, and the female sex hormone concentration after culture is measured. The cultured matter, which has shown degradative activity based on a significantly decreased female sex hormone concentration as an indicator, is transferred several times to the same culture medium, whereby the degrading microorganisms are enriched. The enrichment culture is mixed into a nutrient agar medium or the like, and cultured to form colonies. The resulting colonies are picked up, and cultured in a liquid medium. The harvested resting cells are added to a reaction mixture containing a female sex hormone, and the female sex hormone concentration after reaction is measured. With a significant decrease in this concentration as an indicator, candidate female sex hormone degrading microorganisms are selected. The thus enriched and isolated female sex hormone degrading microorganisms include bacteria of the genus Novosphingobium, the genus Sphingomonas, and the genus Ochrobactrum, or a plurality of bacteria which do not belong to any of them.

From among the bacteria selected by the above-described method, bacteria which can efficiently degrade further desired low concentrations of female sex hormones are selected in the following manner: In selecting them, the female sex hormone is added to the reaction mixture containing these bacterial cells until the female sex hormone reaches a final concentration of 10,000 to 0.001 μg/L. After completion of the reaction, the female sex hormone concentration is measured. With a decrease in the desired concentration of the female sex hormone as an indicator, the preferred female sex hormone degrading bacteria are selected.

In consideration of the lower limit value of the detectable concentration, the amount of the sample necessary for one sampling is set at 400 mL or more at the smallest. Only by so doing can an evaluation be made of the removing ability that can act as far as the very low concentration of 1 ng/L or less. Also can a choice be made of bacteria which are capable of effective degradation treatment in such a very low concentration range.

Publicly known methods, such as a method using an ELISA kit, a method using GC/MS, and a method using LC/MS/MS, can be used as the above method for measuring female sex hormones. Preferred is the LC/MS/MS method which can simultaneously measure female sex hormones such as 17β-estradiol and estrone (see Example 1).

(2) Representative degrading bacteria isolated, and their properties

We isolated five bacterial strains which can efficiently degrade low concentrations of female sex hormones, which were named JEM-1, JEM-2, JEM-3, JEM-14 and JEM-23. Of them, the four strains, JEM-1, JEM-3, JEM-14 and JEM-23, were deposited with International Patent Organism Depositary, National Institute of Advanced Industrial Science and Technology, on February 27, 2003, and were assigned the Accession Nos., FERM P-19234, FERM P-19235, FERM P-19236 and FERM P-19237, respectively. They were deposited in conformity with Budapest Treaty on the international recognition of the deposits of microorganisms and assigned the accession Nos. FERM BP-08637, FERM BP-08638, FERM BP-08639, and FERM BP-08640, respectively. These female sex hormone degrading bacteria were subjected to various tests for investigating their bacteriological properties.

According to studies of the above representative bacteria, the typical properties of the female sex hormone degrading bacteria of the present invention (hereinafter will be referred to as "the present strains or present bacteria") are as follows:

<u>Microbiological properties</u>

Table 1 shows the microbiological properties of the present bacteria.

Table 1 Microbiological properties of female sex hormone degrading bacteria according to the present invention

| | JEM-1 strain | JEM-2 strain | JEM-3 strain | JEM-14 strain | JEM-23 strain |
|---|---|---|---|---|---|
| Cell shape | Rod-shaped<br>0.5~0.6x1.0~1.2 | Rod-shaped<br>0.6~0.7x1.5~2.0 | Rod-shaped<br>0.6~0.7x1.5~2.0<br>Extensible | Rod-shaped<br>0.5~0.6x1.0~1.2 | Rod-shaped<br>0.6x1.2~1.5 |
| Gram staining | - | - | - | - | - |
| Spore forming | - | - | - | - | - |
| Motility | - | + | + | + | + |
| Colony shape | Circular<br>Entire edge<br>smooth<br>Low convex<br>Glossy<br>Light yellow | Circular<br>Entire edge<br>smooth<br>Low convex<br>Glossy<br>Yellow | Circular<br>Entire edge<br>smooth<br>Low convex<br>Glossy<br>Yellow | Circular<br>Entire edge<br>smooth<br>Low convex<br>Glossy<br>Yellow | Circular<br>Entire edge<br>smooth<br>Low convex<br>Glossy<br>Creamy |
| OF test (glucose) | - | - | - | - | Oxidation |
| Attitude to oxygen | Aerobic | Aerobic | Aerobic | Aerobic | Aerobic |
| Nitrate reduction | - | - | - | - | - |
| Indole production | - | - | - | - | - |
| Oxidase | ± | ± | + | + | + |
| Catalase | + | + | + | + | + |
| Aesculin oxidation | ± | + | ± | ± | ± |
| β-Galactosidase | - | + | - | - | - |
| Urea utilization | - | - | - | - | ± |
| Gelatin hydrolysis | - | + | - | + | - |
| Arginine hydrase | - | - | - | - | ± |

Symbols: -; Negative, +; Positive, ±; Weakly positive

Degrading ability for low concentrations of female sex hormones

The present bacteria include bacteria having a significantly higher degrading ability, preferably, a sufficiently higher degrading ability, than do publicly known degrading bacteria, with regard to ethynylestradiol (Table 4 of Example 1). According to the Example, a preferred example of the bacteria having an extremely high ability to degrade ethynylestradiol is JEM-1 strain, and a preferred example of the bacteria having a significantly high ability to degrade ethynylestradiol is JEM-23 strain.

The present bacteria also include bacteria having the ability to efficiently remove estrone, which is prone to remain in biological treated water (see Table 3 of Example 2). According to the Example, preferred examples of the bacteria having a high ability to degrade estrone are JEM-1 strain, JEM-2 strain, JEM-3 strain, JEM-11 strain, JEM-14 strain and JEM-15 strain.

The present bacteria also include bacteria having the ability to degrade female sex hormones having very low concentrations, namely, concentrations lower than 10 μg/L, preferably 10 ng/L, more preferably 1 ng/L. That is, the present bacteria include bacteria which, when 17β-estradiol or estrone is contained at a concentration of 10 μg/L or more in an object to be treated, such as wastewater, effectively degrades at least one of these female sex hormones until a concentration of 10 ng/L or less (1/1000 or less), preferably a concentration of 1 ng/L or less (1/10,000 or less) (see FIGS. 1 and 2 concerned with Example 2).

According to the Example, the present bacteria that are particularly preferred include bacteria which achieve the decrease in the concentration defined above regarding 17β-estradiol and/or estrone within 24 hours, preferably within 3 hours, at their appropriate cell concentration. Examples of the preferred bacteria that achieve such decrease in the concentration within 3 hours are JEM-1 strain, JEM-2 strain, JEM-3 strain, and JEM-14 strain. An example of the preferred bacteria achieving the decrease within 24 hours is JEM-23 strain.

The term "at the appropriate bacterial cell concentration" contemplated herein refers, typically, to about 5 mg/L on an added dry cell weight basis (corresponding to about $5.0 \times 10^7$ CFU/mL)(see Example 2). Incidentally, the above decrease in the concentration can be achieved in a shorter time, for example, within 30 minutes, by further increasing the cell concentration of the present bacteria.

The term "object to be treated" as used herein refers, unless otherwise explained, to wastewater having a proper BOD concentration, its treated wastewater, sludge, or wastes similar to them. If the indication of concentration in a liquid is contemplated, for example, biological treated wastewater is meant.

The present bacteria have also been confirmed to be capable of degrading estriol rapidly as for the above hormones.

Thus, the present bacteria particularly preferred are bacteria having the aforementioned ability to degrade both of 17β-estradiol and estrone, most preferably, all of 17β-estradiol, estrone and estriol.

According to the Example, preferred examples of the bacteria having the effective ability to degrade both of very low concentrations of 17β-estradiol and estrone are JEM-1 strain, JEM-2 strain, JEM-3 strain, JEM-14 strain and JEM-23 strain (see Tables 2 and 3). Preferred examples of the bacteria having the effective ability to degrade all of very low concentrations of 17β-estradiol, estrone and estriol are JEM-1 strain, JEM-2 strain, and JEM-3 strain (see Table 5).

According to the present invention, the use of the present bacteria or every combination of these bacteria, for example, provides a biological treatment method having a preferably improved ability to degrade ethynylestradiol, 17β-estradiol, estrone and estriol.

Degradation rate for low concentrations of female sex hormones

The reaction for degradation by the present degrading bacteria is a first-order reaction, in view of the fact that the degradation rate decreases as the substrate concentration lowers in the substrate concentration range of 10 μg/L or less. Thus, this degradation reaction can be expressed by Equation 1 shown below. The degradation efficiency of the present bacteria can be defined with the use of the degradation rate constant $k_1$ derived from Equation 1.

$$Ln(C/C_0) = k_1 \cdot X \cdot t \qquad \text{Equation 1}$$

where Ln = natural logarithm, C = female sex hormone concentration, $C_0$ = initial female sex hormone concentration, $k_1$ = degradation rate constant (L/(mg·t), X = cell concentration (mg/L), and t = reaction time (h).

To remove 99.9% of the female sex hormones contained in the object to be treated, with the use of 5 mg/L (dry weight) of the degrading microorganism cells, the degradation rate constant $k_1$ of the degrading microorganism for each substance is required to be about 0.5 for a reaction time of 3 hours, and about 0.05 for a reaction time of 24 hours.

The present bacteria include bacteria which, when degrading 17β-estradiol or estrone having a relatively high concentration, such as 10 μg/L, contained in, for example, wastewater, to a concentration of about 10 ng/L or less, preferably to a concentration of about 1 ng/L or less, have a degradation rate constant $k_1$, for the degradation reaction, of 0.05 or more, preferably 0.5 or more (see Table 5 of Example 2, and relevant FIGS. 1 and 2).

More preferably, the present bacteria have a degradation rate constant $k_1$, for each of 17β-estradiol and estrone, of at least 0.05, preferably at least 0.5, and, more preferably, have a degradation rate constant $k_1$, for estrone apt to remain in biological treated water, of 1.5 or more. According to the Example, preferred examples of the bacteria having such a high degradation rate constant $k_1$ are JEM-1 strain, JEM-2 strain, JEM-3 strain, and JEM-14 strain.

According to the present invention, as described above, there are provided bacteria which can degrade, with satisfactory efficiency, 17β-estradiol and/or estrone to such very low concentrations as not to affect ecosystems, such as fish, in aquatic environments. By use of the bacteria, treatment of female sex hormone in a vast amount of wastewater, for example, is provided at a low cost.

<u>Other properties of the present bacteria and culture media, etc.</u>

The respective bacteria were examined for the acid production of various sugars, the assimilability of various substrates, and the enzyme activity (see Tables 6 to 8 of Example 3). In connection with the assimilability, the present bacteria also include bacteria which proliferate using female sex hormones as the single carbon source (see Example 4, FIGS. 4 and 5).

To culture the present bacteria, a culture medium can be used, as long as these bacteria can grow therein. For example, the bacteria can be cultured in culture media utilizing various types of wastewater and female sex hormones, such as 17β-estradiol, as a carbon source, or can be cultured in ordinary nutrient culture media as will be described later.

If the present bacteria can be cultured particularly in nutrient culture media commonly used in microorganism cultivation, for example, peptone, meat extract, and yeast extract, their cultivation and maintenance are easy. This can confer the advantage that the limitations on industrial applicability, which are often encountered with this type of bacteria, are not imposed.

That is, the present bacteria include bacteria having a maximum specific growth rate (μmax), in a nutrient broth medium, of at least 0.1 (see Example 4 and FIGS. 6 to 8). According to the Example, preferred examples of the bacteria showing a preferred specific growth rate in nutrient broth medium are JEM-2 strain, JEM-3 strain, JEM-11 strain, JEM-14 strain, and JEM-23 strain.

The maximum specific growth rate (μmax), as defined herein, means a maximum value of the specific growth rate (μ), which is the function of the amount of change per hour in the cell count measured as OD 660 nm, in shake culture using an optimal culture medium for each of the bacteria. Generally, the maximum specific growth rate refers to the specific growth rate (μ) in the logarithmic growth phase under optimal incubation conditions.

As described above, the present bacteria include not only bacteria which grow on female sex hormones as a single carbon source, but also bacteria which can preferably grow in environments where other abundant carbon sources coexist. In many cases, organic materials of the order of mg/L or more are contained in wastewater to be subjected to biological treatment. According to the present invention, there are also provided strains which degrade low concentrations of female sex hormones efficiently, as described above, even in such environments with abundance of organic matter.

<u>Taxonomic place of the present bacteria</u>

We determined DNA sequence of 16S-rRNA gene of some strains representative of the present bacteria (the DNA sequence of 16S-rRNA gene of JEM-1, JEM-2, JEM-3, JEM-14 and JEM-23 are indicated as Sequence Nos. 1 to 5 of the Sequence Listing). In connection with the DNA sequence of 16S-rRNA gene, analysis was conducted of homology between the present bacteria and various publicly known strains (see Example 3 and FIG. 3).

Herein, the present invention relies on the discovery of the representative strains of the present bacteria and their phylogenetic analysis, and is also directed to providing certain variants capable of having bacteriological properties substantially comparable to those of these representative strains. Hence, the bacteria included in the scope of the present invention can be defined by the following taxonomic places:

① JEM-1 strain is classified under the genus <u>Novosphingobium.</u> Homology analysis of 16S-rRNA shows that JEM-1 strain has homology of 96% with the known female sex hormone degrading bacteria <u>Novosphingobium</u> ARI-1, and has homology of 95%, at the highest, with other related publicly known bacteria.

Thus, the present bacteria include bacteria which have properties comparable to those of JEM-1 strain and belong to the genus Novosphingobium and which have a base sequence more than 95% homologous, preferably more than 96% homologous, and more preferably at least 98% homologous, to the base sequence described in Sequence No. 1 of the Sequence Listing.

② JEM-2 strain is classified under the genus Sphingomonas. Homology analysis of 16S-rRNA shows that JEM-2 strain has homology of 91% with the known female sex hormone degrading bacteria, and has homology of 93%, at the highest, with other related publicly known bacteria.

Thus, the present bacteria include bacteria which have properties comparable to those of JEM-2 strain and belong to the genus Sphingomonas and which have a base sequence more than 91% homologous, preferably more than 93% homologous, and more preferably at least 95% homologous, to the base sequence described in Sequence No. 2 of the Sequence Listing.

③ JEM-3 strain is classified under the genus Sphingomonas. Homology analysis of 16S-rRNA shows that JEM-3 strain has homology of 91% with the known female sex hormone degrading bacteria, and has homology of 93%, at the highest, with other related publicly known bacteria.

Thus, the present bacteria include bacteria which have properties comparable to those of JEM-3 strain and belong to the genus Sphingomonas and which have a base sequence more than 91% homologous, preferably more than 93% homologous, and more preferably at least 95% homologous, to the base sequence described in Sequence No. 3 of the Sequence Listing.

④ JEM-14 strain is classified under the genus Sphingomonas. Homology analysis of 16S-rRNA shows that JEM-14 strain has homology of 92% with the known female sex hormone degrading bacteria, and has homology of 95%, at the highest, with other related publicly known bacteria.

Thus, the present bacteria include bacteria which have properties comparable to those of JEM-14 strain and belong to the genus Sphingomonas and which have a base sequence more than 92% homologous, preferably more than 95% homologous, and more preferably at least 98% homologous, to the base sequence described in Sequence No. 4 of the Sequence Listing.

⑤ JEM-23 strain was classified under the genus Ochrobactrum.

In connection with the genus Ochrobactrum, bacteria having the ability to degrade female sex hormones, such as JEM-23 strain, have not been reported so far. Thus, the present bacteria include bacteria which are characterized by homology with the base sequence described in Sequence No. 5 of the Sequence Listing and belong to the genus Ochrobactrum, and which have properties comparable to those of JEM-23 strain. Examples of such bacteria can include bacteria which have a base sequence at least 88% homologous, preferably at least 94% homologous, and more preferably at least 97% homologous, to the base sequence described in Sequence No. 5 of the Sequence Listing.

Of the above-mentioned bacteria, JEM-2 strain and JEM-3 strain show homology of only 91%, and JEM-14 strain shows homology of only 92%, with the publicly known female sex hormone degrading bacteria ARI-1 strain. In view of these low homologies, it has been concluded that these strains are in new species of the genus Sphingomonas. According to the present invention, there are provided new types of female sex hormone degrading bacteria which have properties comparable to those of JEM-2 strain, JEM-3 strain or JEM-14 strain and belong to the genus Sphingomonas.

(3) Utilization of the present bacteria

According to the present invention, there is provided a method for degradation treatment of a female sex hormone, which comprises bringing female sex hormone degrading bacteria, selected from the present bacteria, with the female sex hormone under aerobic or anaerobic conditions to make the female sex hormone degrading bacteria act on the female sex hormone.

The method of contact may be, but is not limited to, a method of adding the present bacteria or cultures thereof into an object to be treated, the object being contained in a biological treatment reactor; a method of fixing the bacteria or cultures thereof to an immobilization carrier; or a method of adhering or fixing the bacteria or cultures thereof to a filter media to be used in a biological aerated filter process or a rotating biological contactor process.

The object to be treated, to which the present method can be applied, includes various wastewaters, sludge, or wastes similar to them, but they are not restrictive. The wastewater, which can be treated by the present method, typically includes, but is not limited to, domestic wastewater, such as sewage, or stockbreeding wastewater.

The methods of contact with wastewater may be a method of adding the present bacteria into a biological treatment tank; a method of directly adding the present bacteria into wastewater; and a method of charging an immobilization carrier, which has the present bacteria immobilized thereon, into a biological treatment tank, and stably maintaining the bacteria-immobilized carrier within the treatment tank by means of a screen or the like. Alternatively, there can be applied a method comprising passing wastewater through a water-passable container, such as a column, which has been charged with a carrier having the present bacteria carried thereon.

Examples of the carrier having the present bacteria carried thereon are plastics, ceramics, activated carbon, polyacrylamide gel, polyethylene glycol, polyvinyl alcohol, alginic acid, agarose, and dextrin gel. As the method of immobilization, adsorption or entrapment, for example, can be employed. Moreover, the present bacteria can be adhered to and held by a fixing bed of the aerobic filter bed process or the rotating biological contactor process. Furthermore, the present bacteria in free form may be held within a membrane bioreactor, where wastewater may be passed.

As the bacteria for use in the present method, cultures of the bacteria incubated in an ordinary nutrient medium may be used unchanged, or may be used in a refrigerated or frozen form or as dry cells. Moreover, the bacterial cells, which have been harvested and concentrated by centrifugation, etc. of liquid cultures, may be preserved and used, where necessary. Many of the present bacteria, in particular, are advantageous for industrial application, because they can proliferate in the aforementioned ordinary nutrient medium or their growth rate is preferably high.

The final concentration of the present bacteria during their contact with wastewater is, for example, a concentration of $10^3$ cells/L or more, preferably $10^5$/L or more. On a dry weight basis, it is, for example, a concentration of 0.001 mg/L or more, preferably 0.1 mg/L or more.

In the present method, the present bacteria which can degrade at least ethynylestradiol, 17β-estradiol, estrone and estriol are brought into contact with wastewater from which the female sex hormones should be removed, whereby these female sex hormones can be comprehensively removed from the wastewater.

In the present method, moreover, the present bacteria, which have the ability to degrade 17β-estradiol and/or estrone having a concentration of 10 ng/L or less, or 1 ng/L or less, are brought into contact with wastewater from which the female sex hormones should be removed, whereby these female sex hormones can be reliably removed from the wastewater until they reach the desired very low concentrations.

In the present method, moreover, the present bacteria, which have a degradation rate constant $k_1$ for 17β-estradiol and/or estrone of 0.05 or more, preferably 0.5 or more, and more preferably, a degradation rate constant $k_1$ for estrone of 1.5 or more, are brought into contact with wastewater from which the female sex hormones should be removed, whereby these female sex hormones can be efficiently removed from the wastewater within the desired short period of time until the desired very low concentrations are reached.

The female sex hormone concentration detected actually in wastewater is generally of the order of 0.000001 to 0.1 mg/L. According to the present method, the female sex hormone concentration in wastewater can be decreased promptly to less than 10 ng/L, preferably less than 1 ng/L, as stated earlier. No limitation is imposed on the female sex hormone concentration in wastewater to be treated, and this concentration may be 0.1 mg/L or more.

Furthermore, the present method can efficiently remove relatively high concentrations of female sex hormones in sludge by contacting the present bacteria with various sludges or wastewaters under aerobic or anaerobic conditions in a sludge or sludge waste treatment process.

Particularly in a wastewater treatment plant having a sludge treatment process, the female sex hormone concentration in wastewater returned from the sludge treatment step to a wastewater treatment step can be markedly decreased. Thus, the concentration of the female sex hormone present or accumulated in the wastewater treatment system can be decreased. As a result, the female sex hormone concentration in discharged water into the environment can be decreased, and the female sex hormone concentration in sludge to be discarded can be decreased. The possibility of decreasing the female sex hormone concentration in sludge as a waste results in lessening of environmental load encountered when the sludge is restored to farmland.

When the present method is applied to sludge treatment, contact with the present bacteria can be applied to various sludges, such as raw sludge, excess sludge, return sludge, thickened sludge, and anaerobic digested sludge. It is also effective to apply the present bacteria to water separated from sludge, such as filtered water or recycle flow water. For female sex hormone-containing high-concentration wastes, such as human wastes or stockbreeding wastewater, the concentration of the female sex hormones contained therein can be reduced by the same method.

Besides, a female sex hormone degrading agent is provided by use of the present bacteria or cultures of the bacteria. Preferred embodiments of the female sex hormone degrading agent are, but not limited to, liquid cultures and their dry powder. The dry powder, for example, can be prepared by drying treatment using a lyophilization device or the like which is known well in the field of the art. Alternatively, the female sex hormone degrading agent of an entrapped form may be produced by mixing the present bacteria with polymers, such as polyethylene glycol or polyvinyl alcohol, solidifying the mixture, and molding the solids into particles or cubes. Further alternatively, the cultured cells may be brought into contact with the polymer carrier, activated carbon, ceramic carrier or urethane sponge, whereby the cells may be adsorbed thereto. The resulting degrading bacteria-adsorbed carrier can also be prepared and used as a female sex hormone degrading agent.

[Examples]

The present invention will now be described in greater detail by reference to the following Examples.

[Example 1] Isolation of degrading bacteria

Isolation of the bacteria according to the present invention was performed in the following manner:

Preparation of enrichment cultures

Activated sludge was collected from sewage treatment facilities in Tochigi Prefecture, Japan, refrigerated, and transported. Immediately thereafter, the activated sludge was subjected to experiments as seed sludge for enrichment culture. Two culture media, i.e., Yeast Nitrogen Base without amino acid (YNB) medium (Difco), and YNBYE medium consisting of YNB medium and 50 mg/L of yeast extract added thereto, were used as basal media for enrichment culture. To each of the basal media, 17β-estradiol or estrone with a final concentration of 1,000 mg/L was added and used as a carbon source. A solution of each female sex hormone in ethyl acetate was poured into a sterilized test tube. After ethyl acetate was volatilized and removed, the basal medium sterilized by filtration and the seed sludge (final concentration 100 mg/L) were added. The test tube was capped with a silico-stopper, and shake cultured at 28°C. The concentration of 17β-estradiol or estrone after the incubation was measured, and the female sex hormone was regarded as having been degraded, if its concentration was decreased. Out of the enrichment cultures confirmed to show degradation, 1% was transferred to a new medium of the same composition. This procedure was repeated to prepare stable enrichment cultures.

The measurements of 17β-estradiol, estrone and ethynylestradiol were made as follows: The cultures were extracted with 3 mL×2 ethyl acetate into an enrichment culture test tube, and the extract was diluted with 50% (V/V) methanol. An internal standard (17β-estradiol-16,16,17-d3 (Aldrich), estrone-16,16-d2 (CDN Isotopes), or 16α-hydroxy-17β-estradiol-2,4-d2 (CDN Isotopes)) was added, and the mixture was measured with a liquid chromatograph-tandem mass spectrometer (LC/MS/MS). The measurement conditions for LC/MS/MS employed were as follows:

[HPLC]
HPLC: HP1100 (Hewlett Packard), Column: L-column ODS (2.1×150 mm, 5 μm; Chemicals Evaluation and Research Institute, Japan), mobile phase: 40% acetonitrile/water-(2 min)-80% acetonitrile/water, mobile phase flow velocity: 200 μL/min, post-column addition: 0.05% ammonia/methanol; 30 μL/min, amount of sample poured: 20 μL
[MS/MS]
MS/MS: Quattro II (Micromass), ionization mode: ESI (negative), Cone Voltage: 50V, Collision Energy: 38 eV, Collision Gas: argon
Monitor ions: estrone; m/z 269→145, 17β-estradiol; m/z271→145, estriol; m/z287→171, estrone-d2; m/z271→145, 17β-estradiol-d3; m/z274→145, estriol-d2; m/z289→173, ethynylestradiol; m/z295→145

Isolation of female sex hormone degrading bacteria

The enrichment cultures prepared by the above-described method were diluted with sterilized water. The dilution was mixed into an agar medium having 1.5% Bacto Agar (Difco) added to NB medium (Difco) or PGY medium (2 g/L peptone, 0.5 g/L glucose, 1.0 g/L yeast extract, pH 7.0). The solidified agar plate was incubated at 28°C to form colonies. Of the resulting colonies, about 100 were picked up, and measured for female sex hormone degrading activity in the following manner:

NB medium was inoculated with the isolated colonies, and incubated at 28°C by shake culture. The bacterial cells after incubation were centrifuged (5,000 rpm, 15 min) for harvesting, and washed. The washed cells were added to YNB medium containing 1 mg/L of 17β-estradiol or estrone or ethynylestradiol such that the OD at 660 nm would be 1.0. The mixture (5 mL) was poured into a sterilized test tube with a silico-stopper, and reacted at 28°C with shaking. The female sex hormone concentration at Day 0 and Day 7 of the reaction was measured by the same method as described earlier. The microorganisms that showed decreases in the concentrations of the various female sex hormones were selected as female sex hormone degrading bacteria (JEM-1 to JEM-34).

Table 2 shows the results of the 17β-estradiol degradation test. Table 3 shows the results of the estrone degradation test. Table 4 shows the results of the ethynylestradiol degradation test.

In the 17β-estradiol degradation test, the estrone concentration was also measured simultaneously, because many of the degrading bacteria have the ability to oxidize 17β-estradiol to estrone. The controls in these experiments represent the results obtained when treatment was performed without addition of the cells.

Table 2

| 17β-estradiol degradation test (concentration after 1 week of incubation) | | |
|---|---|---|
| Strain | 17β-Estradiol | Estrone |
| | (μg/l) | (μg/l) |
| Control | 1090 | 0 |
| JEM-1 | 0 | 0 |
| JEM-2 | 0 | 5 |
| JEM-3 | 0 | 2 |
| JEM-8 | 0 | 1007 |
| JEM-11 | 55 | 0 |
| JEM-14 | 0 | 0 |
| JEM-15 | 51 | 7 |
| JEM-16 | 20 | 699 |
| JEM-23 | 84 | 331 |
| JEM-29 | 0 | 337 |
| JEM-32 | 37 | 772 |
| JEM-34 | 931 | 126 |

0: Less than the lower limit value of determination (1 μg/l)

Table 3

| Estrone degradation test (concentration after 1 week of incubation) | | |
|---|---|---|
| Strain | 17β-Estradiol | Estrone |
| | (μg/l) | (μg/l) |
| Control | 0 | 998 |
| JEM-1 | 0 | 41 |
| JEM-2 | 0 | 1 |
| JEM-3 | 0 | 41 |
| JEM-8 | 0 | 1011 |
| JEM-11 | 0 | 51 |
| JEM-14 | 0 | 0 |
| JEM-15 | 0 | 0 |
| JEM-16 | 0 | 894 |
| JEM-23 | 0 | 657 |
| JEM-29 | 0 | 553 |
| JEM-32 | 0 | 676 |
| JEM-34 | 114 | 770 |

0: Less than the lower limit value of determination (1 μg/l)

Table 4

| Ethynylestradiol degradation test (concentration after 1 week of incubation) | | |
|---|---|---|
| Strain | Ethynylestradiol | Degradation ratio |
| | (µg/l) | (%) |
| Control | 937 | 6.3 |
| JEM-1 | 63 | 93.7 |
| JEM-2 | 987 | 1.3 |
| JEM-3 | 930 | 7.0 |
| JEM-11 | 960 | 4.0 |
| JEM-14 | 926 | 7.4 |
| JEM-15 | 965 | 3.5 |
| JEM-23 | 853 | 14.7 |

As listed in the above tables, the bacteria isolated in the present tests included bacteria belonging to the genus Novosphingobium (JEM-1), the genus Sphingomonas (JEM-2, JEM-3, JEM-14), and the genus Ochrobactrum (JEM-23), and bacteria belonging to a plurality of genera different from these genera (i.e. JEM-29, JEM-32), in the light of the base sequence information on their 16S-rRNA. Aside from these bacteria, there were also microorganisms (e. g. JEM-8 strain) which oxidize 17β-estradiol to estrone, but show no further degradation of estrone.
The results of Table 4 show that JEM-1 strain had a very high degradation ratio for ethynylestradiol, and JEM-23 strain also showed a significantly high degradation ratio for ethynylestradiol as compared with the control.

[Example 2] Batch treatment test

Female sex hormone batch treatment experiments were conducted in the manner described below. The purposes of the experiments were to select bacteria, which can degrade low concentrations of female sex hormones more efficiently, from among the female sex hormone degrading bacteria obtained as described in Example 1, and to compare their degrading capacity.

Ability to degrade very low concentrations of 17β-estradiol and estrone

The cells incubated in NB medium (Difco) were suspended at a concentration of 5 mg/L in an artificial sewage dilution, and the suspension was innoculated into a 2-liter reactor. To the reactor, 17β-estradiol or estrone was added to a final concentration of 10 µg/L, and a degradation reaction was allowed to proceed at room temperature (25°C) with stirring. The artificial sewage dilution had a composition — 3 mg/L polypeptone, 9.3 mg/L sodium acetate, 0.3 mg/L yeast extract, 2.3 mg/L $MgSO_4 \cdot 7H_2O$, 1.5 mg/L NaCl, 0.9 mg/L $KH_2PO_4$, 0.7 mg/L $CaCl_2 \cdot H_2O$, and 1.5 mg/L $NH_4Cl$ — and had a final BOD of about 7.5 mg/L. The female sex hormone concentrations in the reaction mixture were measured with the passage of time.
The female sex hormone concentration measurement was conducted in the following manner: A certain amount of the reaction mixture was passed through a solid phase extraction column (Sep-pak Plus C18, Waters) with the use of Sep-pak Concentrator (Waters). The column was washed with pure water, and then desiccated, followed by passing methanol therethrough for elution. The eluate was further concentrated, and then water-ether partition recovered an ether phase. After substitution by 50% methanol, an internal standard (17β-estradiol-16,16,17-d3 (Aldrich), estrone-16,16-d2 (CDN Isotopes), or 16α-hydroxy-17β-estradiol-2,4-d2 (CDN Isotopes)) was added, and the mixture was measured by LC/MS/MS. When the amount of the sample taken for one sampling was set at 1 liter, the lower limit value of assay was 0.5 ng/L for 17β-estradiol, and 0.3 ng/L for estrone.
FIGS. 1 and 2 show the results of the above batch treatment test, and represent changes in the concentrations of the respective female sex hormones in low concentration ranges, the changes due to degradation by representative degrading bacteria. These results indicate that JEM-1 strain, JEM-2 strain and JEM-3 strain are able to decrease 10 µg/L of 17β-estradiol and estrone to very low concentrations of the order of 1 ng/L within 3 hours in an environment where easily degradable organic matter coexists.
In the present experiments, all the target bacteria were measured for the 17β-estradiol and estrone concentrations at 30 minutes, 1 hour, 3 hours and 5 hours. In the experimental results shown in FIGS. 1 and 2, however, those concentrations below the lower limit values of detection (0.5 ng/L for 17β-estradiol and 0.3 ng/L for estrone) were not plotted.

As a result of 17β-estradiol degradation by these strains, the formation of estrone associated with decreases in 17β-estradiol was temporarily observed, but the resulting estrone was gradually degraded and decreased, and thus did not remain.

Based on the results of the present test, these bacteria are believed to have the ability to degrade both 17β-estradiol and estrone having very low concentrations.

JEM-14 strain, on the other hand, degraded estrone relatively promptly as did JEM-1 and JEM-3 strains, but with JEM-14 strain, the progress of the degradation reaction rapidly slowed when the estrone concentration reached about 10 ng/L. Neither JEM-16 strain nor JEM-29 strain was nearly able to degrade either hormone within 24 hours.

Ability to degrade estriol

In regard to estriol, the same batch treatment test as described above was performed. The results of the test confirmed the aforementioned representative degrading bacteria to have the ability to degrade estriol promptly.

Table 5 shows the results of the batch treatment test using estriol. JEM-1 strain and JEM-3 strain, in particular, showed the ability to degrade estriol promptly to very low concentrations of less than 10 ng within 3 to 5 hours.

Table 5

| Changes in estriol concentrations in batch treatment test of representative degrading bacteria | | | | |
|---|---|---|---|---|
| Time (h) | JEM-1 | JEM-3 | JEM-14 | JEM-23 |
| 0 | 9972 | 10084 | 10176 | 12650 |
| 1 | 1393 | 1720 | 10154 | 9286 |
| 3 | ND | 3.4 | 10224 | 9339 |
| 5 | ND | ND | 9349 | 9496 |
| 24 | ND | ND | 6899 | 9405 |
| Unit: ng/l, ND: Less than the lower limit value of detection (0.6 ng/l) | | | | |

Degradation rate constant

The degradation rate constants $k_1$'s of the respective degrading bacteria for 17β-estradiol and estrone were calculated from the above-mentioned test results.

Table 6 summarizes the degradation rate constants $k_1$'s for 17β-estradiol and estrone.

Table 6

| The degradation rate constants of the female sex hormone degrading bacteria of the Example | | |
|---|---|---|
| No. No . | 17β-Estradiol degradation rate constant $k_1$ (1/mg·h) | Estrone degradation rate constant $k_1$ (1/mg·h) |
| JEM-1 | 2.074 | 1.725 |
| JEM-2 | 0.684 | 1.742 |
| JEM-3 | 0.581 | 1.905 |
| JEM-8 | E2→E1 only | Not degraded until 24 hours later |
| JEM-11 | 0.607 | 2.056 |
| JEM-14 | 0.453 | 1.87 |
| JEM-23 | 0.079 | 0.060 |

[Example 3] Identification of degrading bacteria

Acid production test. assimilability test. etc.

Tables 7 and 8 show the results of tests for acid production from sugars and for assimilability in connection with the above-described representative strains which efficiently degrade female sex hormones. API20NE (Biomerieux) was used for the assimilability test. API50 (Biomerieux) and API50 CHB medium were used for the acid production test.

Table 7

| Acid production from sugars by the female sex hormone degrading bacteria of the Example | | | | | |
|---|---|---|---|---|---|
| Sugar | JEM-1 strain | JEM-2 strain | JEM-3 strain | JEM-14 strain | JEM-23 strain |
| L-Arabinose | - | - | - | - | + |
| D-Xylose | - | - | - | - | + |
| D-Glucose | - | - | - | - | + |
| D-Mannose | - | - | - | - | + |
| D-Fructose | - | - | - | - | + |
| D-Galactose | - | - | - | - | + |
| Maltose | - | - | - | - | - |
| Sucrose | - | - | - | - | - |
| Lactose | - | - | - | - | - |
| Trehalose | - | - | - | - | - |
| D-Sorbitol | - | - | - | - | + |
| D-Mannitol | - | - | - | - | + |
| Inositol | - | - | - | - | + |
| Glycerol | - | - | - | - | + |
| D-Raffinose | - | - | - | - | - |
| Ribose | - | ± | - | - | + |

- : Negative.
+: Positive.
±: Weakly positive.

Table 8

| Substrate assimilability of the female sex hormone degrading bacteria of the Example | | | | | |
|---|---|---|---|---|---|
| Substrate | JEM-1 strain | JEM-2 strain | JEM-3 strain | JEM-14 strain | JEM-23 strain |
| Glucose | - | - | + | - | + |
| L-Arabinose | - | - | ± | - | + |
| D-Mannose | - | - | + | - | + |
| D-Mannitol | - | - | - | - | + |
| N-Acetyl-D glucosamine | - | - | - | - | + |
| Maltose | - | - | ± | - | - |
| Potassium gluconate | - | ± | + | + | + |
| n-Capric acid | - | - | - | - | + |
| Adipic acid | - | ± | + | - | - |
| dl-Malic acid | - | - | - | - | + |

- : Negative.
+: Positive.
±: Weakly positive.

Table 8 (continued)

| Substrate assimilability of the female sex hormone degrading bacteria of the Example | | | | | |
|---|---|---|---|---|---|
| Substrate | JEM-1 strain | JEM-2 strain | JEM-3 strain | JEM-14 strain | JEM-23 strain |
| Sodium citrate | - | - | - | - | + |
| Phenyl acetate | - | - | - | - | - |

- : Negative.
+: Positive.

Table 9 shows the results of measurements by APIZYM (Biomerieux) for the presence or absence of main enzyme activities in the above bacteria

Table 9

| Enzyme activities of the female sex hormone degrading bacteria | | | | | |
|---|---|---|---|---|---|
| Enzyme | JEM-1 strain | JEM-2 strain | JEM-3 strain | JEM-14 strain | JEM-23 strain |
| Alkaline phosphatase | + | + | + | + | + |
| Esterase (C4) | + | + | + | + | + |
| Esterase lipase (C8) | + | + | + | + | + |
| Lipase (C4) | - | - | - | - | - |
| Leucine allylamidase | + | + | + | + | + |
| Valine allylamidase | + | + | + | + | + |
| Cystine allylamidase | - | + | + | + | + |
| Trypsin | - | + | + | - | + |
| Chymotrypsin | - | - | - | - | - |
| Acid phosphatase | + | + | + | + | + |
| Naphthol-AS-BI-phosphohydrolase | + | + | + | + | + |
| α-Galactosidase | - | - | - | - | - |
| β-Galactosidase | - | - | - | - | - |
| β-glucuronidase | - | + | + | + | - |
| α-Glucosidase | ± | - | - | - | - |
| β-Glucosidase | - | - | + | - | - |
| N-Acetyl-β-glucosamidase | ± | - | + | - | ± |
| α-Mannosidase | - | - | - | - | - |
| α-Fucosidase | - | - | - | - | - |

- : Negative.
+: Positive.
±: Weakly positive.

Homology analysis for DNA sequences of 16S-rRNA genes

The base sequences over nearly the entire length of 16S-rRNA of the above representative strains were determined. The representative DNA sequences are indicated as Sequence No. 1 (JEM-1), Sequence No. 2 (JEM-3), Sequence No. 3 (JEM-14) and Sequence No. 4 (JEM-23) of the Sequence Listing. Further, these base sequences were compared with the sequences of publicly known bacteria, and a phylogenetic tree was prepared by the NJ (neighbor joining) method.
These DNA sequences of 16S-rRNA genes were further analyzed for homology with related existing strains by CLUSTAL W. For the bacteria of the genus Sphingomonas, a new classification method for subdividing this genus into four clusters has been proposed according to International Journal of Systematic and Evolutionary Microbiology (2001), vol. 51, p. 1405. Thus, this new method has been taken into consideration.

FIG. 3 shows a phylogenetic tree representing the place of the present bacteria relative to the publicly known bacteria. Table 10 shows the results of analysis of homology with related existing strains.

Table 10 Homology of 16S-rRNA between the degrading bacteria described in the Example and bacteria of related genera

| Strain | No. | Accession | JEM-1 | JEM-2 | JEM-3 | JEM-14 | JEM-23 |
|---|---|---|---|---|---|---|---|
| Novosphingobium sp. JEM-1 | | | - | 91 | 92 | 93 | 88 |
| Sphingomonas sp. JEM-2 | | | 91 | - | 98 | 94 | 88 |
| Sphingomonas sp. JEM-3 | | | 92 | 98 | - | 94 | 88 |
| Sphingomonas sp. JEM-14 | | | 93 | 94 | 94 | - | 88 |
| S. yanoikuyae | IFO15102 | X85023 | 93 | 93 | 92 | 95 | 87 |
| S. paucimobilis | ATCC29837 | X72722 | 89 | 92 | 92 | 91 | 86 |
| S. chlorophenolica | ATCC33790 | X87161 | 92 | 93 | 93 | 94 | 87 |
| S. asaccharolytica | ATCC51839 | Y09639 | 92 | 93 | 93 | 91 | 87 |
| S. macrogoltabidus | ATCC51380 | D13723 | 94 | 91 | 92 | 95 | 88 |
| S. subarctica | IFO16058 | X94102 | 95 | 91 | 92 | 95 | 88 |
| Novosphingobium sp. AR-1 | | AB070237 | 96 | 91 | 91 | 92 | 88 |
| Ochrobactrum anthropi | | AJ276036 | - | - | - | - | 97 |
| Ochrobactrum grignonense | | AJ242581 | - | - | - | - | 98 |
| Sinorhizobium morelense | | AF452129 | - | | - | - | 94 |

Based on the above study, of the female sex hormone degrading bacteria, JEM-1 strain is classified under the genus Novosphingobium, JEM-2, JEM-3 and JEM-14 strains are classified under the genus Sphingomonas, and JEM-23 strain is classified under the genus Ochrobactrum. (Thus, these strains are also described as Novosphingobium sp. JEM-1 strain, Sphingomonas sp. JEM-2 strain, JEM-3 strain and JEM-14 strain, and Ochrobactrum sp. JEM-23 strain, respectively, and other related strains follow these rules.)

JEM-1 strain is 96% homologous to the existing female sex hormone degrading bacterium ARI-1 strain in terms of the entire length of 16S-rRNA. Moreover, JEM-1 strain has the ability to degrade ethynylestradiol which ARI-1 strain cannot degrade. Thus, JEM-1 strain is a clearly different organism from ARI-1 strain. On the other hand, JEM-2 strain, JEM-3 strain, and JEM-14 have even lower homology with ARI-1 strain of 91%, 91% and 92%, respectively, and are also less homologous to the other known microorganisms, and thus they are new types of bacteria. JEM-23 strain was found to belong to the genus Ochrobactrum.

[Example 4] Growth characteristics of the degrading bacteria

In connection with the above representative bacteria, the growth rate, with 17β-estradiol and estrone as carbon sources, was compared.

The present experiments were conducted by inoculating a test tube having a diameter of 21 mm, capped with a silico-stopper and containing 7 mL of the medium for enrichment culture shown in Example 1, with each of the bacteria at a certain concentration, incubating the inoculum by shake culture at 150 rpm and at 28°C, and measuring changes in OD at 660 nm over time.

FIG. 4 shows growth curves obtained when 17β-estradiol was used as a carbon source, and FIG. 5 shows growth curves obtained when estrone was used as a carbon source.

FIGS. 6 to 8 compare growths in NB medium (Difco) performed similarly. To investigate the influence of temperature, each of the strains was shake cultured at 15, 25, 30, 35 and 40°C, and the time courses of OD660nm were measured. Based on the results, each of the strains showed the highest growth rate at 35°C.

JEM-1 strain in the NB medium showed the same level of the growth rate as when using 17β-estradiol or estrone as the carbon source, while JEM-3 strain and JEM-14 strain showed higher growth rates with the use of the NB medium. These bacteria were also confirmed to multiply even in general nutrient media, such as PGY medium or trypticasesoy medium, nearly similarly to NB medium.

Table 11 shows the maximum specific growth rate (μmax) of each of the bacteria incubated at 35°C in NB medium, the values of μmax being calculated based on the above results. This table also includes the results obtained by similar methods for a plurality of other degrading bacteria.

Table 11

| Specific growth rates of the female sex hormone degrading bacteria of the Example | | |
|---|---|---|
| Strain | μmax (h-1) | Genus |
| JEM-1 | 0.03 | Novosphingobium sp. |
| JEM-2 | 0.29 | Sphingomonas sp. |
| JEM-3 | 0.33 | Sphingomonas sp. |
| JEM-8 | - | - |
| JEM-11 | 0.38 | Sphingomonas sp. |
| JEM-14 | 0.37 | Sphingomonas sp. |
| JEM-23 | 0.46 | Ochrobactrum sp. |

μmax: Maximum specific growth rate

[Example 5] Wastewater treatment using degrading bacteria

Batch treatment of female sex hormones with the use of degrading bacteria was performed using inflow sewage, biological treated water, gravity thickened concentrated sludge, an aerobic digested sludge and filtered water of sewage treatment plants, and further stockbreeding wastewater.

The present experiments were performed by stirring 2 liters of each wastewater placed in a wide-mouthed glass bottle by means of a magnetic stirrer, adding each of the degrading bacteria at a concentration of 5 mg/L, and stirring the mixture at room temperature (25°C) to carry out the reaction. After 2 hours of reaction, 1 liter of each sample was taken, and the female sex hormone concentration was measured in the manner described below.

The method of pretreatment for measuring the concentrations of 17β-estradiol, estrone and estriol in the wastewater samples by LC/MS/MS was as follows: Each sewage sample was filtered through a glass filter, and then an internal standard was added, with 500 mL of raw water or 1,000 mL of treated water set as the standard volume. Then, the mixture was concentrated by solid phase extraction. The residue on the filter was extracted in methanol by application of ultrasonic waves, and the extract was mixed with the filtrate. The sludge sample was separated into solids and a liquid by centrifugation (3,000 rpm, 15 min), and then the precipitate was extracted with acetate buffered methanol and methanol in this sequence. The extract was concentrated by a rotary evaporator, whereafter the concentrate was mixed with the supernatant, and mixture was concentrated by solid phase extraction as in the case of the liquid sample. The solid phase column used was Oasis HLB Plus (Waters). After passage of the sample, the column was washed with ultrapure water and 30% acetone in this order, desiccated, and then eluted with methanol. The eluate was concentrated in a nitrogen gas stream, and distributed by water-ether partition, and then the ether phase was recovered. Furthermore, cleanup was performed using a Sep-pak NH2 (Waters) cartridge, and estrone and 17β-estradiol were recovered into the ether eluate, while estriol was recovered into the methanol eluate. The recovered substances were each concentrated, redissolved, and then measured by LC/MS/MS. The operating conditions for LC/MS/MS are as described in Example 1.

Table 12 shows the concentrations of 17β-estradiol and estrone in wastewater treated for 2 hours after addition of the present bacteria. The results confirmed that the concentration of each female sex hormone after 2 hours of reaction was markedly decreased in any treated water incorporating the present bacteria. In the system similarly reacted for 2 hours without the addition of the present bacteria, no decrease in the female sex hormone was observed in any wastewater.

Table 12 Results of treatment of female sex hormone-containing wastewater by the degrading bacteria of the Example

| Sample | Control | | | JEM-1 treated water | | | JEM-3 treated water | | |
|---|---|---|---|---|---|---|---|---|---|
| | E1 (ng/l) | E2 (ng/l) | E3 (ng/l) | E1 (ng/l) | E2 (ng/l) | E3 (ng/l) | E1 (ng/l) | E2 (ng/l) | E3 (ng/l) |
| Inflow sewage | 35.4 | 18.5 | 344.8 | 0.8 | <0.6 | <1.0 | 1.7 | <0.6 | 261.0 |
| Biological treated water | 15.1 | 0.9 | <1.0 | <0.3 | <0.6 | <1.0 | <0.3 | <0.6 | <1.0 |
| Filtered water of sludge | 164 | 1.2 | - | <0.3 | <0.6 | - | <0.3 | <0.6 | - |
| An aerobic digested sludge | 240 | 6.8 | - | 83 | <0.6 | - | 26 | <0.6 | - |
| Stock-breeding wastewater | 1268 | 1524 | - | 24 | <6.0 | - | 19 | 46.0 | - |

E1: Estrone. E2: 17β-estradiol. E3: Estriol. -: Not measured.

[Example 6] Effect of degrading bacteria addition to activated sludge

Activated sludge (MLSS: 1,500 mg/L) of wastewater treatment facilities for treating domestic wastewater was collected, and JEM-1 strain or JEM-3 strain was added thereto at concentrations of 1, 5 and 20 mg/L. Further, 17β-estradiol was added such that its final concentration would be 10 μg/L. The mixture was subjected to aeration for 1 hour, and then centrifuged (3,000 rpm, 15 min). The concentrations of 17β-estradiol and estrone in the supernatant were measured.

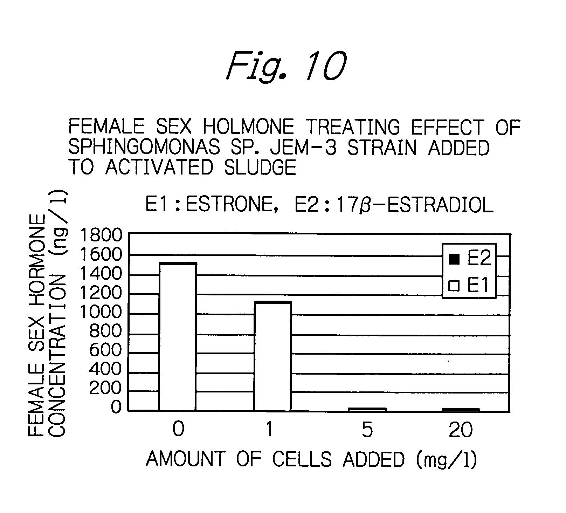

FIG. 9 shows the treating effect of JEM-1 strain, and FIG. 10 shows the treating effect of JEM-3 strain.

In this activated sludge, most of the remaining female sex hormone was converted into estrone upon 1 hour of reaction. Even in this case, a clear treating effect was produced by adding the degrading bacteria at a concentration of 1 mg/L (dry weight) or higher.

As described above, the present invention provides bacteria which degrade 17β-estradiol, estrone, estriol and/or ethynylestradiol with high efficiency. By utilizing these bacteria, these female sex hormones in wastewater and wastes can be removed to such low concentrations that their adverse influence on ecosystems in environments, such as rivers and lakes, can be fully diminished.

While the present invention has been described in the foregoing fashion, it is to be understood that the invention is not limited thereby, but may be varied in many other ways. Such variations are not to be regarded as a departure from the spirit and scope of the invention, and all such modifications as would be obvious to one skilled in the art are intended to be included within the scope of the appended claims.

## Claims

1. Female sex hormone degrading bacteria belonging to a genus Novosphingobium, a genus Sphingomonas or a genus Ochrobactrum and having at least one of the following properties (a) to (c):

   (a) Having an ability to degrade at least ethynylestradiol among female sex hormones;
   (b) Having an ability to degrade at least 17β-estradiol and/or estrone among female sex hormones, and having a degradation rate constant $k_1$ for 17β-estradiol and/or estrone of 0.05 or more; and
   (c) Having an ability to degrade at least 17β-estradiol and/or estrone among female sex hormones, and having an ability to decrease a concentration of 17β-estradiol and/or estrone, contained in an object to be treated, to less than 10 ng/L.

2. The bacteria according to claim 1, which have the property (a) and have an ability to further degrade 17β-estradiol, estrone and estriol.

3. The bacteria according to claim 1, which have the property (b), and have a degradation rate constant $k_1$ for 17β-estradiol and/or estrone of 0.5 or more.

4. The bacteria according to claim 1, which have the property (c) and have the ability to decrease the concentration of 17β-estradiol and/or estrone from about 10 μg/L to less than 10 ng/L.

**5.** The bacteria according to claim 4, which have the property (c) and have the ability to decrease the concentration to about 1 ng/L further.

**6.** The bacteria according to claim 4 or 5, which have the property (c) and have an ability to achieve the concentration decrease in 24 hours, at most, at a proper cell concentration thereof.

**7.** The bacteria according to claim 4 or 5, which have the property (c) and have an ability to achieve the concentration decrease in 3 hours, at most, at a proper cell concentration thereof.

**8.** The bacteria according to any one of claims 1 to 7, which have a maximum specific growth rate (μmax) of at least 0.1 when cultured in a nutrient medium.

**9.** The bacteria according to claim 1, which are Novosphingobium sp. JEM-1 strain (FERM BP-08637).

**10.** The bacteria according to claim 1, which are Sphingomonas sp. JEM-3 strain (FERM BP-08638) or Sphingomonas sp. JEM-14 strain (FERM BP-08639).

**11.** The bacteria according to claim 1, which are Ochrobactrum sp. JEM-23 strain (FERM BP-08640).

**12.** A method for degradation treatment of a female sex hormone, which comprises bringing the bacterial according to any one of claims 1 to 11, or a carrier having the bacteria immobilized thereon, into contact with the female sex hormone.

**13.** The method according to claim 12, which is a method for degradation treatment of a female sex hormone in wastewater, and which comprises passing the wastewater through a reactor including a carrier having the bacteria according to any one of claims 1 to 11 carried thereon.

**14.** The method according to claim 12 or 13, wherein the female sex hormone to be removed is ethynylestradiol, 17β-estradiol, estrone and/or estriol.

**15.** A female sex hormone degrading agent containing the bacteria according to any one of claims 1 to 11, or cultures of the bacteria.

Fig. 1

CHANGES IN 17β-ESTRADIOL CONCENTRATIONS IN BATCH TREATMENT EXPERIMENTS BY REPRESENTATIVE DEGRADING BACTERIA

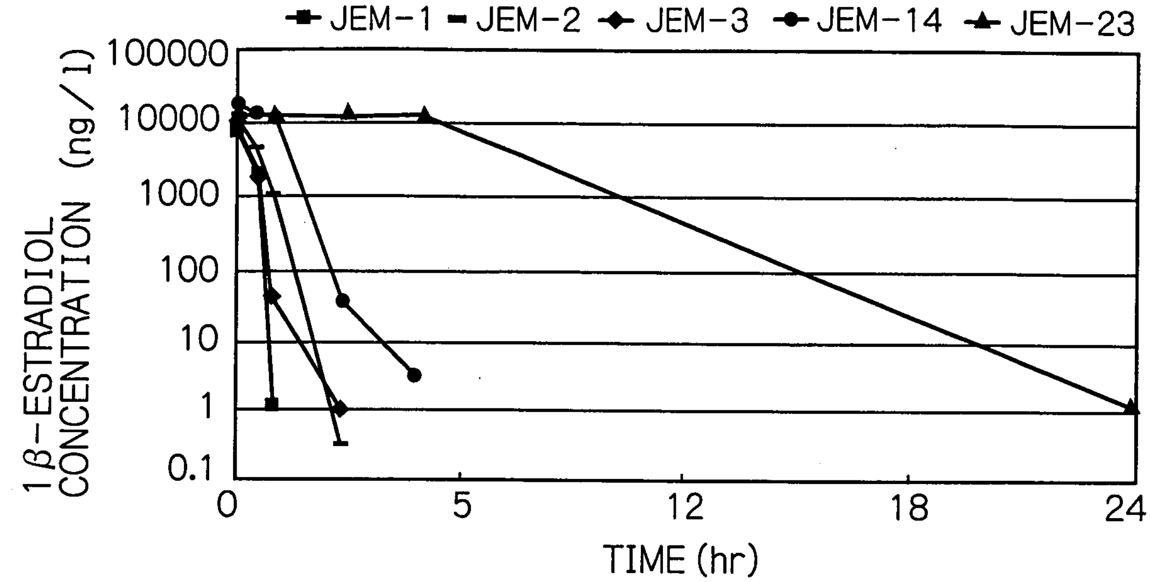


Fig. 2

CHANGES IN ESTRONE CONCENTRATIONS IN BATCH TREATMENT EXPERIMENTS BY REPRESENTATIVE DEGRANDING BACTERIA

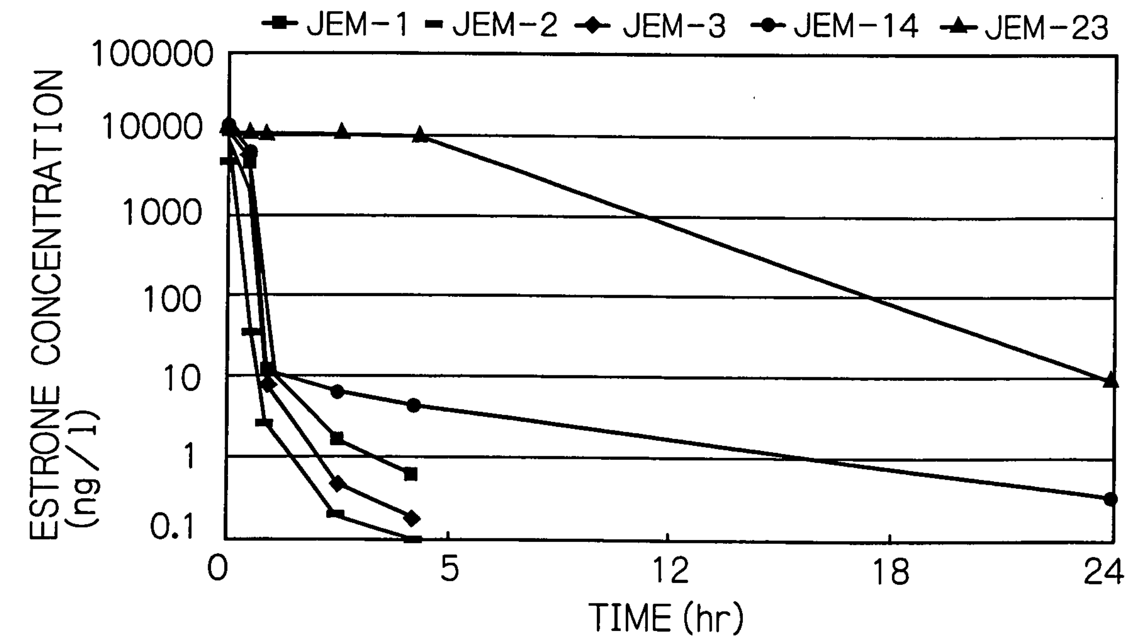

# *Fig. 3*

PHYLOGENETIC TREE PREPARED BY COMPARING DNA SEQUENCES OF THE 16s・rRNA GENES OF FEMALE SEX HORMONE DEGRADING MICROORGANISMS AND PUBLICLY KNOWN RELATED MICROORGANISMS

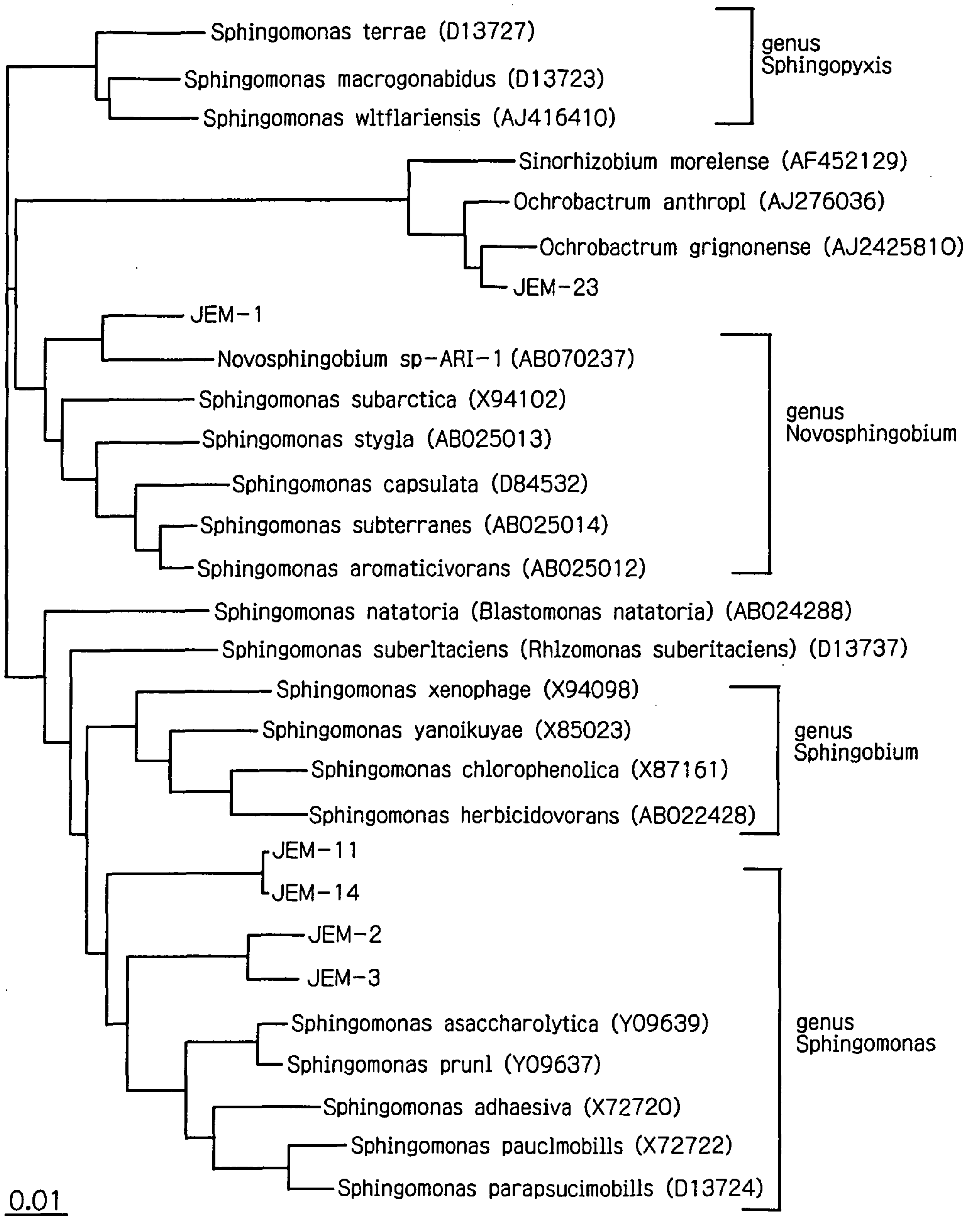

*Fig. 4*

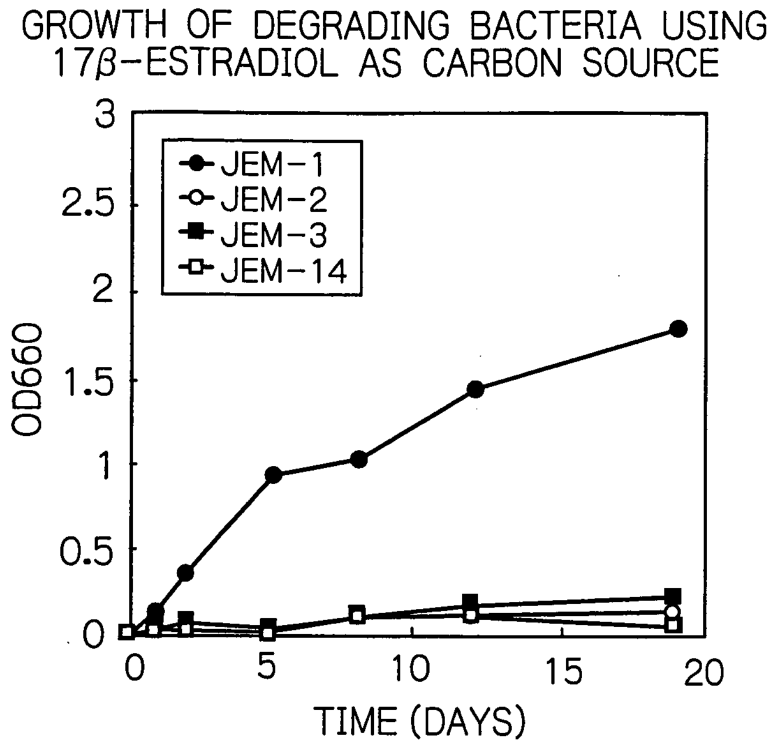
GROWTH OF DEGRADING BACTERIA USING
17β-ESTRADIOL AS CARBON SOURCE
JEM-1
JEM-2
JEM-3
JEM-14
OD660
3
2.5
2
1.5
1
0.5
0
0
5
10
15
20
TIME (DAYS)

*Fig. 5*

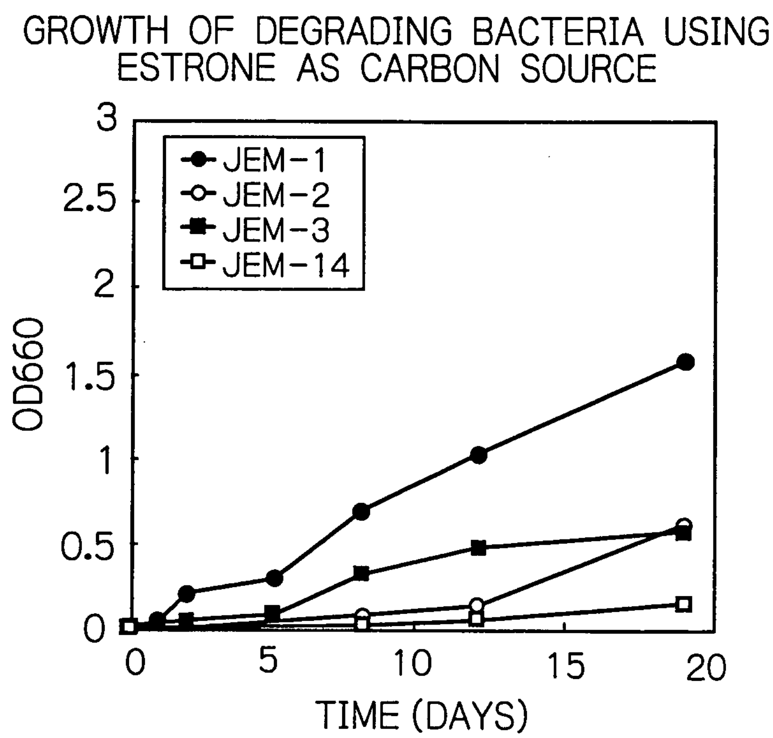
GROWTH OF DEGRADING BACTERIA USING
ESTRONE AS CARBON SOURCE
JEM-1
JEM-2
JEM-3
JEM-14
OD660
3
2.5
2
1.5
1
0.5
0
0
5
10
15
20
TIME (DAYS)

Fig. 6

GROWTH CURVES OF NOVOSPHINGOBIUM SP. JEM-1 STRAIN AT RESPECTIVE TEMPERATURES

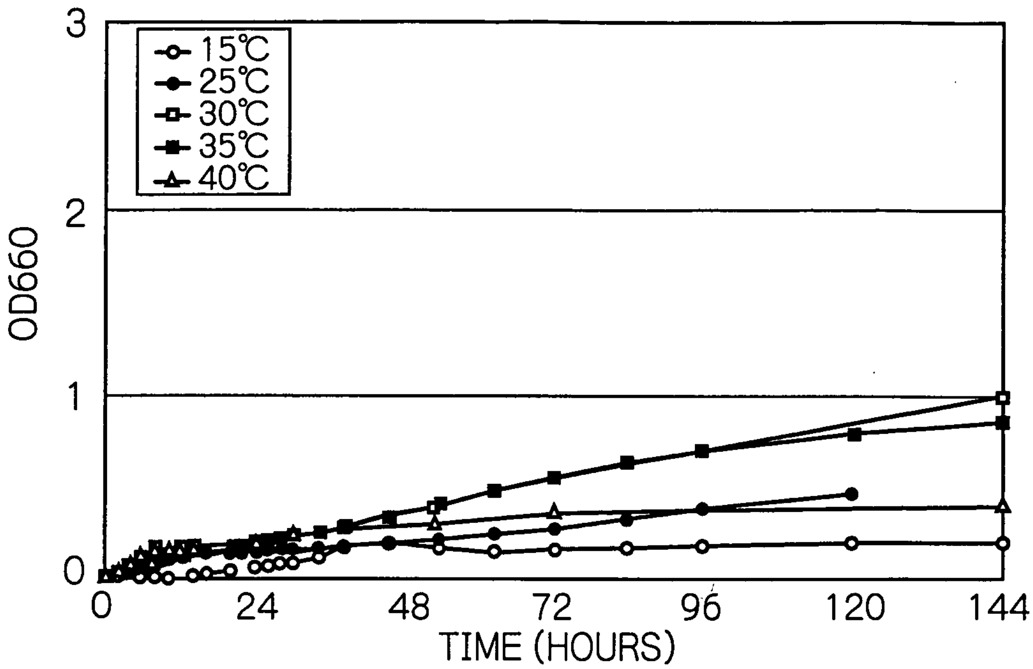


Fig. 7

GROWTH CURVES OF SPHINGOMONAS SP. JEM-3 STRAIN AT RESPECTIVE TEMPERATURES

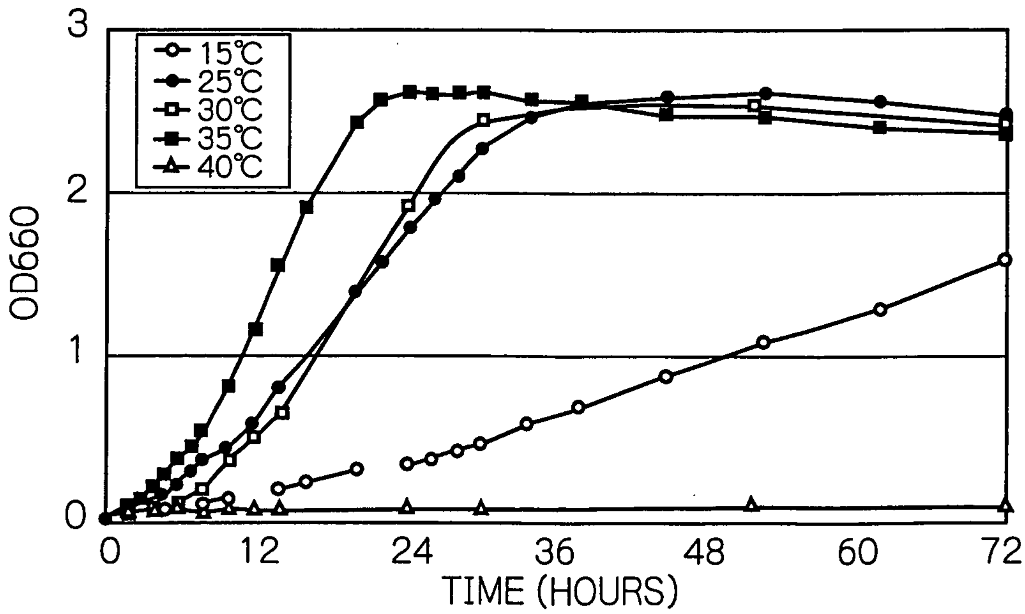

Fig. 8

GROWTH CURVES OF OCHROBACTRUM SP. JEM-23 STRAIN AT RESPECTIVE TEMPERATURES

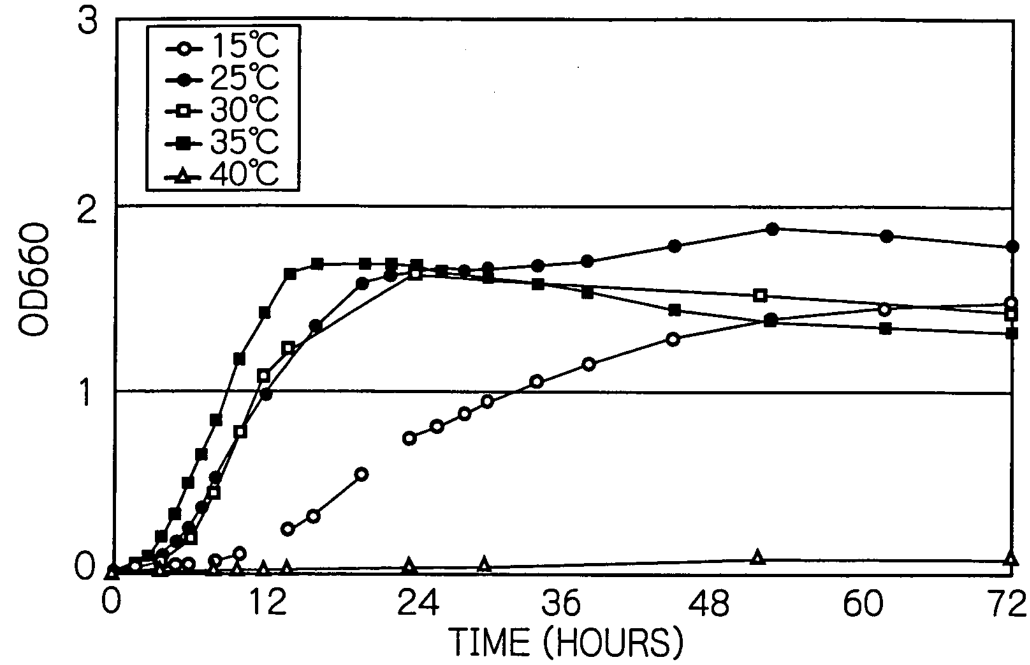


Fig. 9

FEMALE SEX HOLMONE TREATING EFFECT OF NOVOSPHINGOBIUM SP. JEM-1 STRAIN ADDED TO ACTIVATED SLUDGE

E1 : ESTRONE, E2 : 17β-ESTRADIOL

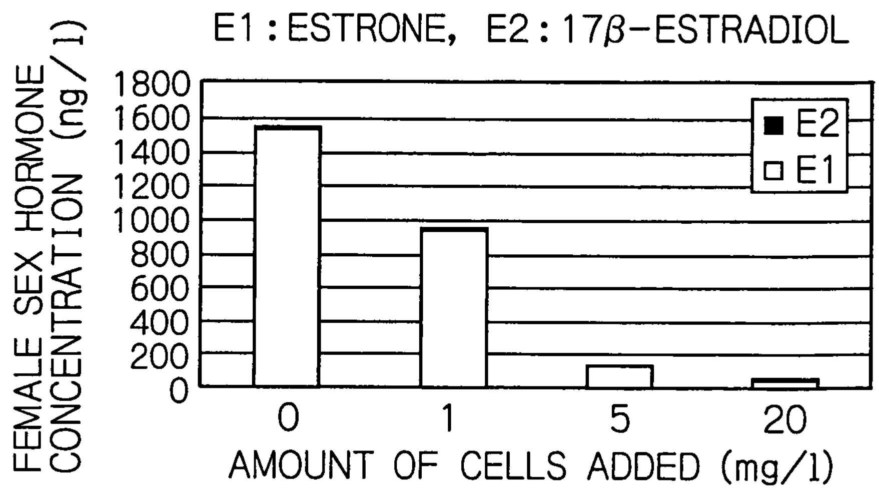

*Fig. 10*

FEMALE SEX HOLMONE TREATING EFFECT OF SPHINGOMONAS SP. JEM-3 STRAIN ADDED TO ACTIVATED SLUDGE